# EUROPEAN PATENT APPLICATION

(11) **EP 4 582 069 A1**
(43) Date of publication of application: **09.07.2025**
(21) Application number: 24220593.8
(22) Date of filing: 17.12.2024
(51) Int. Cl.: A61F 13/15, B07C 5/36, B65B 57/00, G01N 21/00, G05B 19/418

(54) **ROBOTIC AUTOMATION OF PRODUCT QUALITY ASSURANCE METHODS**

(30) Priority: 03.01.2024 US 202463617212 P
(71) Applicant: The Procter & Gamble Company, Cincinnati, OH 45202 (US)
(72) Inventor: SCHNEIDER, Uwe, 45202 Cincinnati (US); RAYCHECK, Jeromy Thomas, 45202 Cincinnati (US); SHEEHAN, Astrid Annette, 45202 Cincinnati (US)
(74) Representative: P&G Patent Germany

(57) **Abstract**

A method is provided for making absorbent articles on an absorbent article manufacturing line. The method includes the steps of advancing a continuous substrate and adding parts and other substrates to the continuous substrate to form a continuous length of absorbent articles. The method includes the step of separating discrete absorbent articles from the continuous length of absorbent articles to form a stream of discrete absorbent articles. The method includes the step of advancing the stream of discrete absorbent articles to a packaging apparatus. The method includes the step of automatically diverting some absorbent articles from the stream to a robotic inspection station. The method includes the step of automatically inspecting the diverted absorbent articles using the robotic inspection station.

## Description

### CROSS REFERENCE TO RELATED APPLICATION

This application claims the benefit of U.S. Provisional Patent Application No. 63/617,212, filed on January 3, 2024, the entire disclosure of which is incorporated herein by reference.

### FIELD

The present disclosure relates generally to quality assurance methods for product manufacturing and more specifically to robotic automation of quality assurance methods for product manufacturing.

### BACKGROUND

Current absorbent article production processes are complex, combining as many as 30 different raw materials at very high processing speeds. As a consequence, small changes in process conditions or material properties can result in product performance degradation. Currently several hundred individual quality assurance methods are being deployed to confirm good production quality. While some of these methods are simple visual inspections that can be performed while the product is made (for example by a camera), other methods are more complex and can only be performed after the product is finished. These tests may be performed by the line team (on a quality table near the converter) or in a central lab. Such methods may for example include destructive testing (such as elastic force measurements) or weight measurements (such as core sectional weights). One drawback of these conventional methods is the manual effort of quality inspections to be performed after the product is made is significant and hence costly.

The discussion of shortcomings and needs existing in the field prior to the present disclosure is in no way an admission that such shortcomings and needs were recognized by those skilled in the art prior to the present disclosure.

### SUMMARY

Various aspects solve the above-mentioned problems and provide methods and devices useful for robotic automation of inspections of product manufacturing processes. The disclosure herein overcomes the noted drawbacks of conventional manual inspection by providing robotic automation of such inspections that can reduce manual labor and associated cost, and at the same time reduce the amount of "human error" as robots are generally regarded as more repeatable than humans for simple repetitive tasks.

Additionally, the disclosure overcomes the noted drawback of some conventional methods which are limited to inspection of manufactured products after packaging. This is due to the relatively fast speed of production which exceeds a speed of manual inspection. In some aspects of the disclosure, a repository is provided for individually manufactured products prior to packaging and thus individually manufactured products can be inspected from the repository. This saves significant waste and costs of packaging a defectively manufactured product by avoiding the additional steps of opening the packaged defectively manufactured product and discarding it potentially along with properly manufactured products in the same package.

Furthermore, it is hereby recognized that methods for manufacturing absorbent articles must be relatively fast in order to be commercially viable. In conventional methods, this necessarily requires that the manufacturing equipment be positioned in an arrangement where the manufactured product is not readily accessible at different stages of manufacturing for inspection. In the disclosure herein it was realized that positioning an inspection station at a different vertical level than the manufacturing line facilitates access to the manufactured product at various stages of the manufacturing for inspection. As a result, the disclosure herein discusses various aspects where inspection of the manufactured product can be readily performed by accessing different stages of the manufacturing line which was not possible with conventional methods.

In one aspect of the disclosure, a method is provided for making absorbent articles on an absorbent article manufacturing line. The method includes the steps of advancing a continuous substrate and adding parts and other substrates to the continuous substrate to form a continuous length of absorbent articles. The method includes the step of separating discrete absorbent articles from the continuous length of absorbent articles to form a stream of discrete absorbent articles. The method includes the step of advancing the stream of discrete absorbent articles to a packaging apparatus. The method includes the step of automatically diverting some absorbent articles from the stream to a robotic inspection station. The method includes the step of automatically inspecting the diverted absorbent articles using the robotic inspection station.

These and other features, aspects, and advantages of various embodiments will become better understood with reference to the following description, figures, and claims.

### BRIEF DESCRIPTION OF THE DRAWINGS

Many aspects of this disclosure can be better understood with reference to the following figures.
FIG. 1 is an example according to various aspects illustrating a block diagram of a system for making absorbent articles on an absorbent article manufacturing line;
FIG. 2 is an example according to various aspects illustrating a marking placed on a characteristic of an absorbent article manufactured by the system of FIG. 1;
FIG. 3A is an example according to various aspects illustrating an imaging device of an inspection unit of the robotic inspection station in FIG. 1;
FIG. 3B is an example according to various aspects illustrating an electronic image of an absorbent article generated by the imaging device of FIG. 3A;
FIG. 4 is an example according to various aspects illustrating the absorbent article manufacturing line and the robotic inspection station of FIG. 1 located at the same manufacturing facility;
FIG. 5 is an example according to various aspects illustrating the absorbent article manufacturing line and the robotic inspection station of FIG. 1 located at different facilities;
FIG. 6 is an example according to various aspects illustrating a label of a product code placed on an absorbent article manufactured by the system of FIG. 1;
FIG. 7 is an example according to various aspects illustrating a block diagram of repositories for absorbent articles and packages between the absorbent article manufacturing line and robotic inspection station of FIG. 1;
FIG. 8 is an example according to various aspects illustrating a block diagram of a system for making absorbent articles on an absorbent article manufacturing line;
FIG. 9 is an example according to various aspects illustrating a flow chart depicting steps of a method for making absorbent articles on an absorbent article manufacturing line;
FIG. 10 is an example according to various aspects illustrating a block diagram of a computer system upon which an aspect of the disclosure may be implemented;
FIG. 11 is an example according to various aspects illustrating a block diagram of a chip set upon which an aspect of the disclosure may be implemented; and
FIG. 12 is an example according to various aspects illustrating a block diagram of a mobile terminal for communications which is capable of operating in the system of FIG. 1.

It should be understood that the various embodiments are not limited to the examples illustrated in the figures.

### DETAILED DESCRIPTION

### Introduction and Definitions

This disclosure is written to describe the invention to a person having ordinary skill in the art, who will understand that this disclosure is not limited to the specific examples or embodiments described. The examples and embodiments are single instances of the invention which will make a much larger scope apparent to the person having ordinary skill in the art. Unless defined otherwise, all technical and scientific terms used herein have the same meaning as commonly understood by the person having ordinary skill in the art. It is also to be understood that the terminology used herein is for the purpose of describing examples and embodiments only, and is not intended to be limiting, since the scope of the present disclosure will be limited only by the appended claims.

All the features disclosed in this specification (including any accompanying claims, abstract, and drawings) may be replaced by alternative features serving the same, equivalent, or similar purpose, unless expressly stated otherwise. Thus, unless expressly stated otherwise, each feature disclosed is one example only of a generic series of equivalent or similar features. The examples and embodiments described herein are for illustrative purposes only and that various modifications or changes in light thereof will be suggested to the person having ordinary skill in the art and are to be included within the spirit and purview of this application. Many variations and modifications may be made to the embodiments of the disclosure without departing substantially from the spirit and principles of the disclosure. All such modifications and variations are intended to be included herein within the scope of this disclosure. For example, unless otherwise indicated, the present disclosure is not limited to particular materials, reagents, reaction materials, manufacturing processes, or the like, as such can vary. It is also to be understood that the terminology used herein is for purposes of describing particular embodiments only and is not intended to be limiting. It is also possible in the present disclosure that steps can be executed in different sequence where this is logically possible.

All numeric values are herein assumed to be modified by the term "about," whether or not explicitly indicated. The term "about" generally refers to a range of numbers that one of skill in the art would consider equivalent to the recited value (for example, having the same function or result). In many instances, the term "about" may include numbers that are rounded to the nearest significant figure.

In everyday usage, indefinite articles (like "a" or "an") precede countable nouns and noncountable nouns almost never take indefinite articles. It must be noted, therefore, that, as used in this specification and in the claims that follow, the singular forms "a," "an," and "the" include plural referents unless the context clearly dictates otherwise. Thus, for example, reference to "a support" includes a plurality of supports. Particularly when a single countable noun is listed as an element in a claim, this specification will generally use a phrase such as "a single." For example, "a single support."

Where a range of values is provided, it is understood that each intervening value, to the tenth of the unit of the lower limit (unless the context clearly dictates otherwise), between the upper and lower limit of that range, and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

In this specification and in the claims that follow, reference will be made to a number of terms that shall be defined to have the following meanings unless a contrary intention is apparent.

"Disposed on" refers to a positional state indicating that one object or material is arranged in a position adjacent to the position of another object or material. The term does not require or exclude the presence of intervening objects, materials, or layers.

"Absorbent article" refers to devices that absorb and contain liquid, and more specifically, refers to devices that are placed against or in proximity to the body of the wearer to absorb and to contain various exudates discharged from the body. Examples absorbent articles comprise taped diapers, pants, adult incontinence products, sanitary napkins, tampons, and/or liners.

### System

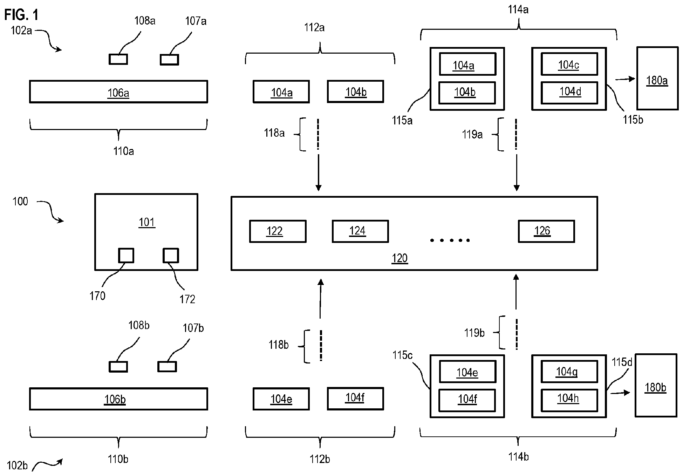

FIG. 1 is an example according to various aspects illustrating a block diagram of a system 100 for making absorbent articles 104 on an absorbent article manufacturing line 102. In one aspect of the disclosure, multiple absorbent article manufacturing lines 102a, 102b are provided in the system 100. However, in other aspects of the disclosure only one absorbent article manufacturing line is provided. Although FIG. 1 depicts two absorbent article manufacturing lines 102a, 102b in other embodiments more than two absorbent article manufacturing lines are provided in the system 100.

The formation of the absorbent articles by the absorbent article manufacturing line 102 will now be discussed. In an aspect of the disclosure, each absorbent article manufacturing line 102a, 102b features a continuous substrate 106a, 106b. In one example aspect, the continuous substrate is one or more components of the absorbent article or diaper disclosed in U.S. Patent No. 7,371,302 B2 and U.S. Pat. Appl. Pub. No. 2023/0157901A1.

In another aspect of the disclosure, one or more parts or other substrates are added to the continuous substrate 106a, 106b to form a continuous length 110a, 110b of absorbent articles. In one example aspects, the added parts include structural components of the absorbent article and/or added substrates, such as those disclosed in U.S. Patent No. 7,371,302 B2 and U.S. Pat. Appl. Pub. No. 2023/0157901A1.

As shown in FIG. 1, in one aspect of the disclosure the continuous length 110a of absorbent articles are separated into discrete absorbent articles 104a, 104b that form a stream 112a of discrete absorbent articles. The continuous length 110b of absorbent articles is similarly separated into discrete absorbent articles 104e, 104f to form a stream 112b of discrete absorbent articles. In an example aspect, this separation is performed with a cutting device, as appreciated by one of ordinary skill in the art.

The packaging of the discrete absorbent articles will now be discussed. The stream 112a, 112b of discrete absorbent articles are advanced to a packaging apparatus 114a, 114b where the discrete absorbent articles are packaged. As shown in FIG. 1, in one aspect of the disclosure the packaging apparatus 114a receiving the stream 112a of discrete absorbent articles secures the discrete absorbent articles 104a, 104b into a first package 115a and additional discrete absorbent articles 104c, 104d of the stream 112a into a second package 115b. Similarly, in one aspect of the disclosure the packaging apparatus 114b receiving the stream 112b of discrete absorbent articles secures the discrete absorbent articles 104e, 104f into a first package 115c and additional discrete absorbent articles 104g, 104h of the stream 112b into a second package 115d. Any suitable number of absorbent articles may be present in a package, such as 24, 48, or 100, for example.

A robotic inspection station 120 of the system will now be discussed that is used to automatically inspect some of the discrete absorbent articles in the stream and/or the packages. In one aspect of the disclosure some of the discrete absorbent articles 104 in the stream and/or the packages 115 formed by the absorbent article manufacturing line 102 are diverted to the robotic inspection station 120. In one example aspect, discrete absorbent articles 104 are diverted to the robotic inspection station 120 upstream of the packaging apparatus 114 and thus the diverted discrete absorbent articles 104 are not positioned in a package 115. Similarly, in another example aspect, packages 115 are diverted to the robotic inspection station 120 downstream of the packaging apparatus 114 such that the diverted absorbent articles 104a, 104b are positioned within the package 115a.

In one aspect of the disclosure, those discrete absorbent articles 104 in the stream 112 that are not diverted to the robotic station are sent to the packaging apparatus 114 and placed in a package 115. Furthermore, in this aspect of the disclosure, those packages 115 that are not diverted to the robotic inspection station 120 are sent to the end 180 of the absorbent article manufacturing line 102. However, in other aspects of the disclosure the robotic inspection station 120 is part of the absorbent article manufacturing line 102 and thus no diversion of the discrete absorbent articles 104 or packages 115 would be necessary for inspection.

In some aspects of the disclosure, the discrete absorbent articles 104 and/or the packages 115 are diverted to the robotic inspection station 120 based on a time based cycle. In this example aspect of the disclosure, the discrete absorbent articles 104 and/or the packages 115 are diverted to the robotic inspection station 120 at a particular rate such as a particular diversion rate based on time (e.g., about 1 absorbent article or package per five minutes of run time to about 1 absorbent article or package per fifty hours of run time). In this example aspect of the disclosure, this rate can be adjusted based on data obtained during the inspection of the discrete absorbent articles 104 and/or packages 115 by the robotic inspection station 120.

In other aspects of the disclosure, the discrete absorbent articles 104 and/or the packages 115 are diverted to the robotic inspection station 120 based on an absorbent article or package frequency. In this example aspect of the disclosure, the discrete absorbent articles 104 and/or the packages 115 are diverted to the robotic inspection station 120 at a particular rate such as a diversion rate based on a rate of absorbent articles or packages (e.g., about 1 absorbent article 104 is diverted per 100 absorbent articles in the stream 112 and/or about 1 package 115 is diverted per 100 packages passing through the packaging apparatus 114). In this example aspect of the disclosure, this rate can be adjusted based on data obtained during the inspection of the discrete absorbent articles 104 and/or packages 115 by the robotic inspection station 120.

In yet other aspects of the disclosure, the discrete absorbent articles 104 and/or the packages 115 are diverted to the robotic inspection station 120 based on an event based cycle. In this example aspect of the disclosure, the discrete absorbent articles 104 and/or the packages 115 are diverted to the robotic inspection station 120 based on the occurrence of one or more events during the manufacture of the absorbent articles 104 and/or packages 115. As shown in FIG. 1, in these embodiments, a sensor 107 is provided in the absorbent article manufacturing line 102 that measures a value of one or more parameters related to the manufacture of the absorbent articles 104 and/or packages 115. In one example aspect, the sensor 107 comprises a camera or imaging device that is configured to capture image data during the manufacture of the absorbent articles 104 and/or packages 115 to determine a value of one or more dimensions of the continuous substrate 106, discrete absorbent articles 104 and/or packages 115. In another example aspect, the sensor 107 comprises a temperature sensor that is configured to measure temperature data during the manufacture of the absorbent articles 104 and/or packages 115 to determine a value of a temperature of the continuous substrate 106, discrete absorbent article 104 and/or packages 115 or a value of a temperature of one or more of the components on the absorbent article manufacturing line 102. In these example aspects, the data from the sensor 107 (e.g., image data and/or temperature data) is transmitted to a controller 101 which then determines whether or not the sensor data is within an acceptable range for each parameter (e.g., stored in a memory 172 of the controller 101). If the sensor data is not within the acceptable range, the controller 101 identifies the occurrence of an event for purposes of the event based cycle. In some example aspects, upon the occurrence of a single event and/or a trend of the occurrence of a particular event (e.g., more than a certain number of events occurring within a certain period of time), the controller 101 transmits a signal to divert the absorbent articles 104 and/or packages 115 to the robotic inspection station 120. The inventors recognized that this event based cycle advantageously ensures that the absorbent articles 104 and/or packages 115 are diverted during the occurrence of undesired events during the manufacture of the absorbent articles 104 and/or packages 115. Event based diverting of the absorbent articles 104 and/or packages 115 may also occur after manufacturing line stops, manufacturing line pauses, and/or material splices, for example, to ensure product quality remains within specifications after these events.

Although a camera and temperature sensor are discussed herein, in other aspects the sensor 107 can comprise any sensor that measures the value of any parameter related to the manufacture of the absorbent articles 104 and/or packages 115. Additionally, although FIG. 1 depicts the sensor 107 at the continuous substrate 106 portion of the absorbent article manufacturing line 102, in other aspects the sensor 107 can be positioned at any location within the absorbent article manufacturing line 102. In still other aspects, the sensor 107 could be positioned within the robotic inspection station 120 and thus would generate sensor data that can be used to determine the occurrence of an event based on diverted absorbent articles 104 and/or packages 115 within the robotic inspection station 120 (e.g., dimensions of one or more diverted absorbent articles 104 not being within an acceptable range).

As shown in FIG. 1, in some aspects of the disclosure the diverted discrete absorbent articles 104 are diverted to a repository 118 that is downstream of the stream 112 of discrete absorbent articles and upstream of the robotic inspection station 120. Similarly, in other aspects of the disclosure the diverted packages 115 are diverted to a repository 119 that is downstream of the packaging apparatus 114 and upstream of the robotic inspection station 120. The repositories 118, 119 advantageously permit the diversion of the discrete absorbent articles 104 and/or packages 115 despite the speed of manufacture of the discrete absorbent articles 104 and/or packages 115 being greater than the speed of inspection with the robotic inspection station 120. Although FIG. 1 depicts two repositories 118a, 118b to receive the diverted discrete absorbent articles 104 from the multiple manufacturing lines 102a, 102b in other aspects one repository 118 is provided that receives the diverted discrete absorbent articles 104 from the multiple manufacturing lines 102a, 102b and subsequently transports these discrete absorbent articles 104 to the robotic inspection station 120. Similarly, although FIG. 1 depicts two repositories 119a, 119b to receive the diverted packages 115 from the multiple manufacturing lines 102a, 102b in other aspects one repository 119 is provided that receives diverted packages 115 from the multiple manufacturing lines 102a, 102b and subsequently transports these packages 115 to the robotic inspection station 120. In this example aspect of the disclosure, the diverted absorbent articles 104 and/or packages 115 remain at the respective repository 118, 119 until the robotic inspection station 120 has available space to receive the diverted absorbent articles 104 and/or packages 115.

As shown in FIG. 1, in an aspect of the disclosure the robotic inspection station 120 includes one or more inspection units 122, 124, 126 which each perform a different inspection of the diverted discrete absorbent articles and/or packages. Although three inspection units are depicted in FIG. 1, in other embodiments the robotic inspection station 120 includes less or more than three inspection units. In one example aspect of the disclosure, one or more inspection units 122, 124, 126 perform a first test (e.g., force measurement test, weight measurement test, etc.). In yet another example of the disclosure, one or more inspection units 122, 124, 126 perform a second test (e.g., softness assessment test, perfume presence test, etc.). In some aspects of the disclosure, multiple different inspections are performed on the same diverted absorbent article 104 and/or package 115 using multiple inspection units 122, 124, 126. In other aspects, some of the inspection units 122, 124, 126 are exclusively used with one of the diverted absorbent articles 104 or packages 115.

In an example aspect, one of the inspection units that receives the diverted packages 115 includes components that open the package 115 in order to access the discrete absorbent articles 104 within the package 115. In some example aspects, after opening the package 115 this inspection unit can assess one or more characteristics of the package (e.g., check for holes, test for strength, etc.). In this example aspect, the robotic inspection station 120 comprises components that are configured to open the package 115 and subsequently remove the discrete absorbent articles 104 within the package 115. The robotic inspection station 120 can then inspect the package 115 and/or the discrete absorbent articles 104 that were removed from the package 115.

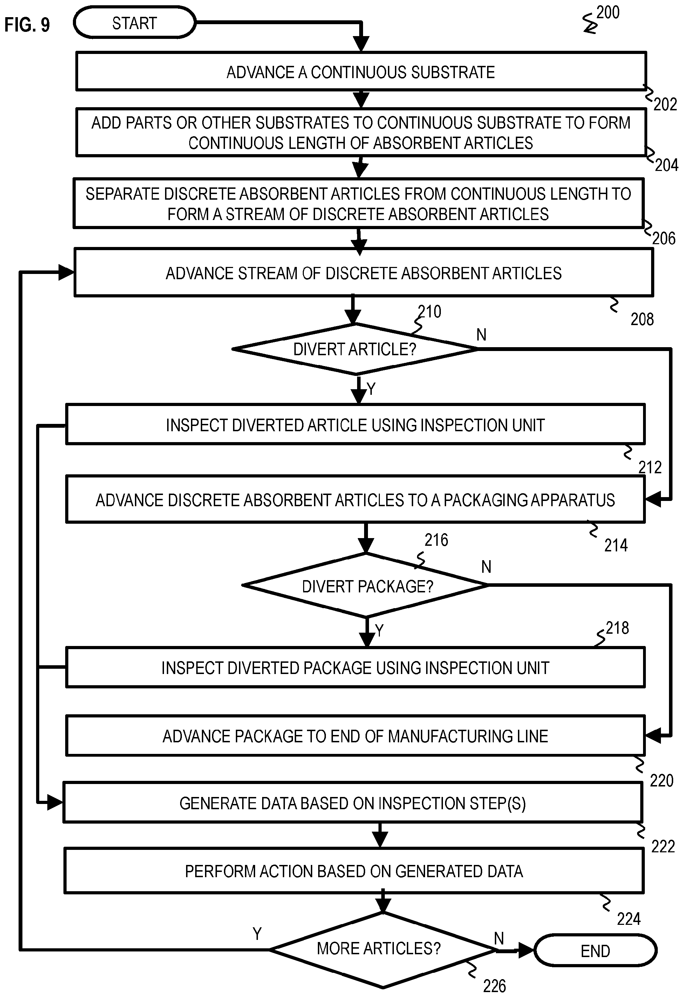

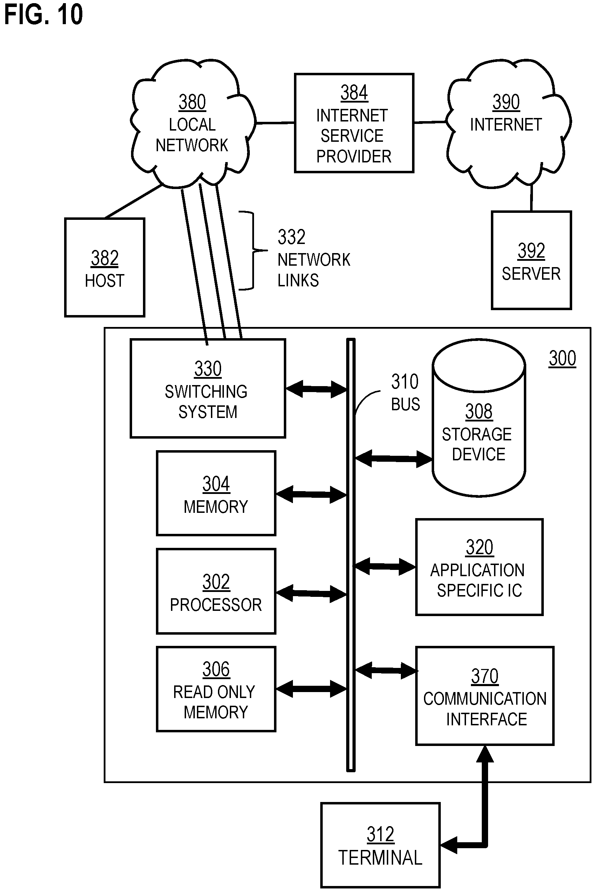

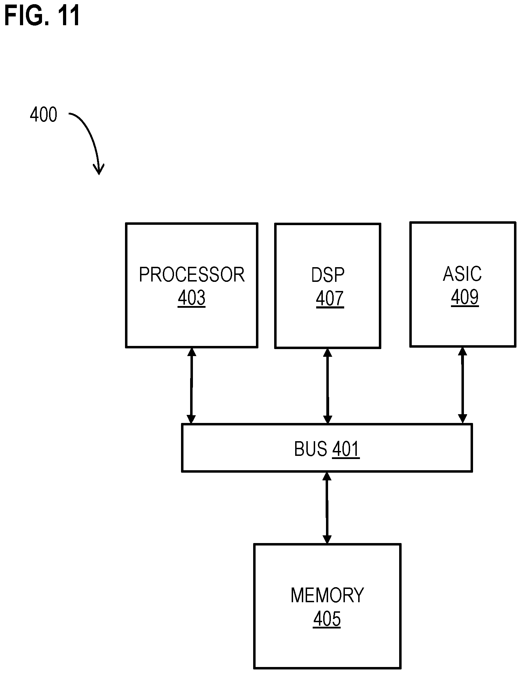

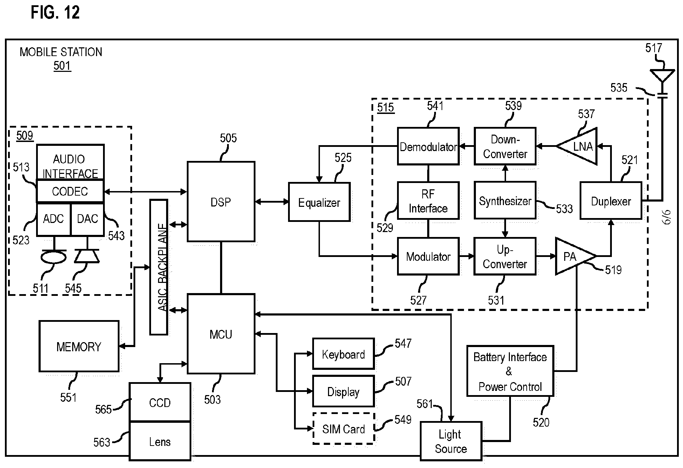

As shown in FIG. 1, in an aspect of the disclosure the controller 101 is provided that is communicatively coupled with the absorbent article manufacturing line 102 and the robotic inspection station 120. In one aspect of the disclosure, the controller 101 is communicatively coupled with one or more components of the absorbent article manufacturing line 102 including an interface 108 (e.g., display) to communicate information to workers on the absorbent article manufacturing line 102; one or more transport mechanisms (e.g., conveyors) that move the continuous length 110 of absorbent articles to the cutting device where the discrete absorbent articles 104 are cut and/or that move the discrete absorbent articles 104 to the packaging device 114 and/or that move the packages 115 to the end of the manufacturing line 180. Additionally, in some aspects the controller 101 is communicatively coupled to transport mechanisms (e.g., conveyors) that divert the discrete absorbent articles 104 and/or the packages 115 (e.g., via the repositories 118, 119) to the robotic inspection station 120. The controller 101 is also communicatively coupled with the robot inspection station 120 including to the one or more inspection units 122, 124, 126 and is configured to receive data from the inspection units based on the inspection of the diverted discrete absorbent articles 104 and/or packages 115. In one example aspect, the controller 101 includes a memory 172 to store the received data from the robotic inspection station 120. In various aspects, the controller 101 includes an inspection module 170 with instructions to cause the system and controller 101 to perform one or more steps of the method 200 of FIG. 9. In some aspects, the controller 101 comprises a general purpose computer system, as depicted in FIG. 10 or a chip set as depicted in FIG. 11 or a mobile terminal as depicted in FIG. 12.

In some aspects of the disclosure, the interface 108 is provided at each absorbent article manufacturing line 102. In an example aspect, the interface 108 includes a transmission device/system for real time access of an operator (e.g., line leader, engineer on-call, plant manager, etc.) to communicate data based on the inspection of the diverted discrete absorbent articles 104 and/or packages 115. In this example aspect, the interface 108 includes a display to output data including the results of the inspection and/or trends of the inspection so that certain action can be taken by the operator of the absorbent article manufacturing line 102 (e.g., stop the manufacturing line 102, adjust one or more parameters of the manufacturing line 102 such as speed, adjust a rate of diversion of the absorbent articles and/or packages to the robotic inspection station 120, investigate a particular component of one of the manufacturing lines that is causing a defect in one of the diverted absorbent articles and/or packages, etc.) In some example aspects, the interface 108 is a mobile device that is used by the operator of the absorbent article manufacturing line 102.

In other aspects of the disclosure, rather than outputting data on the interface 108 (e.g., display) to cause a human operator to take corrective action based on the results of the inspection, the controller 101 can transmit one or more signals to automatically take corrective action based on the results of the inspection. In one example aspect, based on the results of the inspection, the controller 101 transmits a signal to a component of the absorbent article manufacturing line 102 to either adjust one or more operating parameters of the component or to shut down the component. In these example aspects, the controller 101 identifies a specific component and a specific parameter of the component which needs adjusting based on the particular results of the inspection (e.g., where the controller 101 recognizes a trend in excessive temperature on the continuous substrate 106a and/or a trend of excessive dimensions on a particular portion of the absorbent articles 104, etc.).

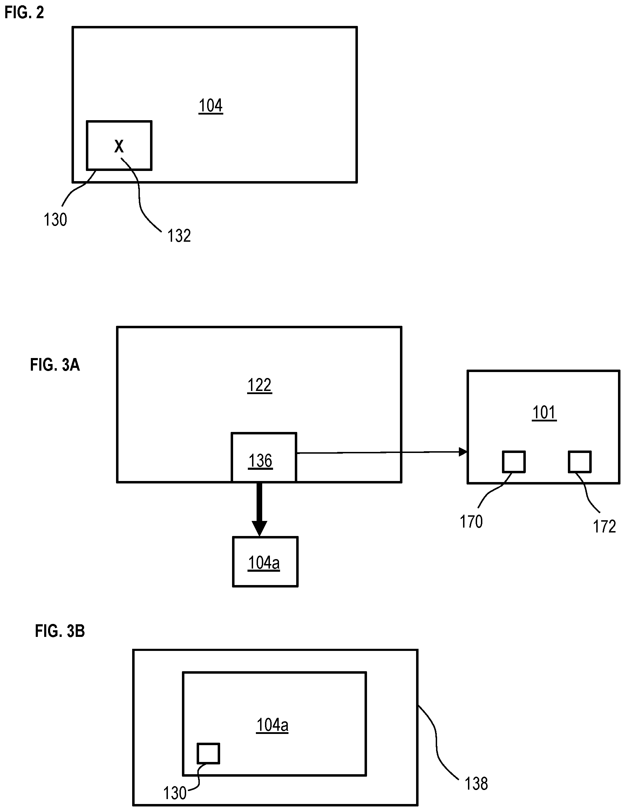

Some aspects of the inspection performed by the robotic inspection station 120 will now be discussed. In one aspect of the disclosure, the robotic inspection station 120 assessed one or more characteristics 130 of the discrete absorbent article 104. In this aspect of the disclosure, upon identifying the one or more characteristics 130 of the discrete absorbent article 104, the robotic inspection station 120 can place a mark or label on the one or more characteristics 130. FIG. 2 is an example according to various aspects illustrating a marking 132 placed on the characteristic 130 of an absorbent article 104 manufactured by the system 100 of FIG. 1. In some aspects, the robotic inspection station 120 includes a printer to print the label or marking 132 which is then placed on the characteristic 130 of the absorbent article 104. In one example aspect, the marking 132 is a quality control (QC) sticker or certification to certify that the characteristic 130 of the absorbent article 104 either does or does not conform with QC criteria. In some example aspects, upon generating the marking 132 the absorbent article 104 can be sent to operators of the manufacturing line 102 and/or to a QC department for the manufacturing line 102 for subsequent action (e.g., discarding the absorbent article 104 if the marking 132 indicates non-conformance with QC criteria, investigating the manufacturing line 102 which generated the absorbent article 104 to remedy any components of the manufacturing line 102 responsible for non-conformance with QC criteria, etc.).

In other aspects of the disclosure, not only does the robotic inspection 120 inspect for the presence of the one or more characteristics, the robotic inspection station 120 inspects the one or more characteristics 130 to assess whether they conform with predetermined criteria (e.g., QC criteria). In one example aspect, the characteristic 130 is a detected defect in the discrete absorbent article 104 or package 115. In still another example aspect, the characteristic 130 is a trend in the identification of detected defects in the diverted discrete absorbent articles 104 and/or packages 115.

In some aspects, the robotic inspection station 120 inspects for the one or more characteristics 130 that are a positive aspect of the discrete absorbent articles 104, such that the robotic inspection station 120 inspects for the existence of the characteristic 130. In this example aspect, the positive aspect of the characteristic 130 may result in either a positive (pass) or a negative (fail) result of the inspection, depending on whether the identified characteristic should be present on the discrete absorbent article 104. Similarly, with the diverted packages 115 the robotic inspection station 120 assesses for characteristics 130 that are positive aspects of the diverted package 115 (e.g., a characteristic which is present on the diverted package). Additionally, identification of such positive aspect characteristic in the diverted package 115 may result in a positive (pass) or negative (fail) result of the inspection depending on whether the characteristic 130 should be present in the diverted package 115.

In some aspects, the robotic inspection station 120 inspects for the one or more characteristics 130 that are a negative aspect of the discrete absorbent articles 104, such that the robotic inspection station 120 inspects for the non-existence of the characteristic 130. In this example aspect, the negative aspect of the characteristic 130 may result in either a positive (pass) or a negative (fail) result of the inspection, depending on whether the identified characteristic should not be present on the discrete absorbent article 104. Similarly, with the diverted packages 115 the robotic inspection station 120 assesses for characteristics 130 that are negative aspects of the diverted package 115 (e.g., a characteristic which is not present on the diverted package). Additionally, identification of such negative aspect characteristic in the diverted package 115 may result in a positive (pass) or negative (fail) result of the inspection depending on whether the characteristic 130 should not be present in the diverted package 115.

In some aspects, the memory 172 of the controller 101 stores data indicating the positive and negative aspect characteristics of the diverted absorbent articles 104 and packages 115 and thus the controller 101 determines the result of the inspection by comparing the inspection data received from the robotic inspection station 120 with the data stored in the memory 172 indicating the positive and negative aspects of the characteristics. In an example aspect, where the controller 101 receives data from the robotic inspection station 120 indicating a positive aspect of a characteristic 130 in the discrete absorbent article 104 (e.g., hole in the discrete absorbent article 104) the controller 101 compares this data with the data in the memory 172 which indicates that this positive aspect characteristic should be present on the absorbent article 104 and thus a positive (pass) inspection result is issued. In an example aspect, where the controller 101 receives data from the robotic inspection station 120 indicating a negative aspect of a characteristic 130 in the discrete package 115 (e.g., hole not present in the package 115) the controller 101 compares this data with the data in the memory 172 which indicates that this negative aspect characteristic should be present on the package 115 and thus a negative (fail) inspection result is concluded.

Imaging of the diverted absorbent articles 104 and/or packages 115 by the robotic inspection station 120 will now be discussed. FIG. 3A is an example according to various aspects illustrating an imaging device 136 of the inspection unit 122 of the robotic inspection station 120 in FIG. 1. As shown in FIG. 3A, in an aspect of the disclosure the inspection unit 122 includes the imaging device 136 to capture images of the diverted discrete absorbent article 104a. The generated image data from the imaging device 136 is then transmitted to the controller 101 which processes the image data in order to assess whether the discrete absorbent article 104a conforms with one or more predetermined criteria (e.g., QC criteria). FIG. 3B is an example according to various aspects illustrating an electronic image 138 of the absorbent article 104a generated by the imaging device 136 of FIG. 3A. In an example aspect, the image 136 includes the characteristic 130 of the discrete absorbent article 104a. In this example aspect, the controller 101 processes the image 138 and identifies the characteristic 130 of the discrete absorbent article 104a and determines a positive (pass) or negative (fail) result of the inspection depending on whether the identified characteristic 130 corresponds with predetermined criteria (e.g., QC criteria) stored in the memory 172 of the controller 101.

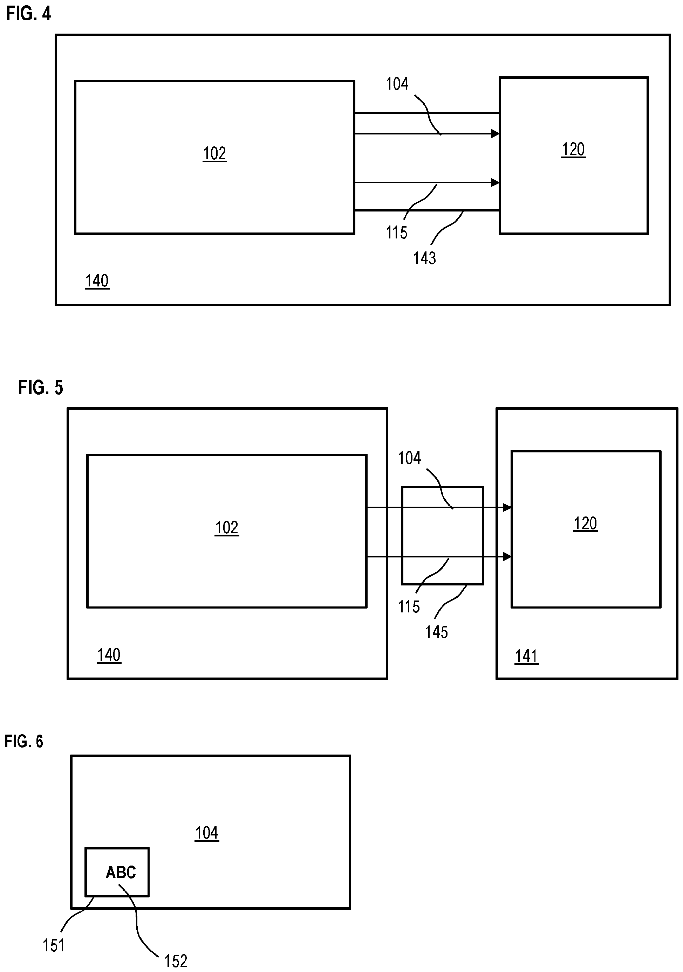

The location of the absorbent article manufacturing line 102 and the robotic inspection station 120 will now be discussed. In some aspects of the disclosure, the absorbent article manufacturing line 102 and the robotic inspection station 120 are located at the same manufacturing facility. FIG. 4 is an example according to various aspects illustrating the absorbent article manufacturing line 102 and the robotic inspection station 120 of FIG. 1 located at the same manufacturing facility 140 (e.g., on the same floor of a building, in different floors of the same building, in different buildings at the same facility, etc.). In this aspect of the disclosure, the absorbent article manufacturing line 102 and the robotic inspection station 120 are located adjacent to each other such that the diverted absorbent articles 104 and/or packages 115 are moved from the manufacturing line 120 to the robotic inspection station 120 using a conduit 143 (e.g., simple conveyor). In other aspects of the disclosure, the absorbent article manufacturing line 102 and the robotic inspection station 120 may be sufficiently close to each other (e.g., across the street from each other, in the same building but in different floors, etc.) so that the conduit 143 (e.g., high speed air tunnel) is used to divert the absorbent articles 104 and/or packages 115 across short distances. This advantageously permits the robotic inspection station 120 to perform the necessary inspections of the diverted absorbent articles 104 and/or packages 115 from a number of different locations relative to the absorbent article manufacturing lines 102. This provides design flexibility in terms of positioning of the robotic inspection station 120 relative to multiple absorbent article manufacturing lines 102.

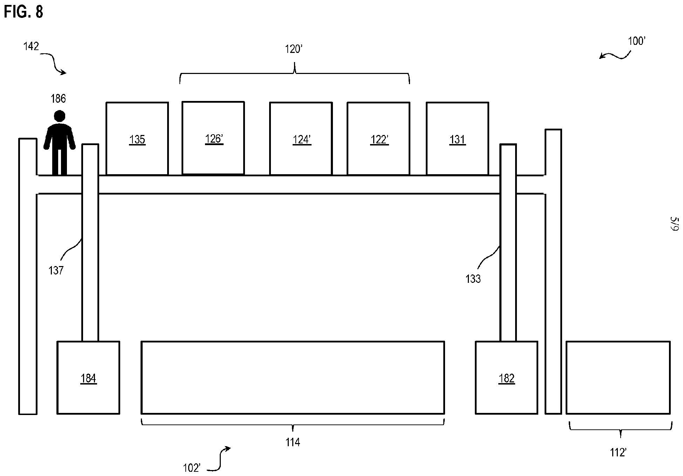

In other aspects of the disclosure, the absorbent article manufacturing line 102 and the robotic inspection station 120 are positioned at different floors at a same location. FIG. 8 depicts an example aspect with this type of arrangement and will be discussed in greater detail below.

In other aspects of the disclosure, the absorbent article manufacturing line 102 and the robotic inspection station 120 are positioned at different locations. FIG. 5 is an example according to various aspects illustrating the absorbent article manufacturing line 102 and the robotic inspection station 120 of FIG. 1 located at different facilities 140, 141. In one aspect of the disclosure, a vehicle 145 is provided to transport the diverted absorbent articles 104 and/or packages 115 from the manufacturing facility 140 where the absorbent article manufacturing line 102 is located to the inspection facility 141 where the robotic inspection station 120 is located. In one example aspect of the disclosure, the inspection facility 141 is operated by a third party to whom the inspection of the diverted absorbent articles 104 and/or packages 115 is outsourced. In another example aspect of the disclosure, the facilities 140, 141 are operated by the same entity and the diverted absorbent articles 104 and/or packages 115 from multiple manufacturing facilities 140 are diverted to the same single inspection facility 141.

In some aspects of the disclosure, each diverted absorbent article 104 and/or package 115 includes a product code which is used during the inspection step. FIG. 6 is an example according to various aspects illustrating a label 151 of a product code 152 placed on an absorbent article 104 manufactured by the system 100 of FIG. 1. In other aspects, the diverted packages 115 feature a similar label with a product code. As appreciated by one skilled in the art, the product code provides various information regarding the manufacturing of the absorbent article 104 or package 115 including one or more of which manufacturing line 102 it was made on, the time and date it was made, the names of the operators of the manufacturing line 102 when it was made, the components of the manufacturing line 102 which made it, etc. In these aspects of the disclosure, the robotic inspection station 120 (e.g., imaging device 136) reads the product code 152 of the diverted absorbent article 104 and/or package 115 and transmits data indicating the product code 152 to the controller 101. In this aspect, the controller 101 then performs one or more actions depending on the result of the inspection of the diverted absorbent article 104 and/or package 115. In an example aspect of the disclosure, if the result of the inspection is negative (fail), the controller 101 processes the data indicating the product code 152 to determine information regarding the manufacturing of the diverted absorbent article and/or package. In this example aspect, upon determining that the diverted absorbent article 104 was manufactured on the absorbent article manufacturing line 102b in FIG. 1, the controller 101 transmits a signal to output on the interface 108b (e.g., display) at the absorbent article manufacturing line 102b various data regarding the defectively manufactured absorbent article 104 and/or package 115 (e.g., time and date it was made, which components of the absorbent article manufacturing line 102b was used, which operator was on duty at the time it was made, etc.). The operator of the absorbent article manufacturing line 102b can then take various actions based on viewing this data on the interface 108b (e.g., shut down the manufacturing line 102b, investigate the one or more components of the manufacturing line 102b that were used to make the defective absorbent article, vary the rate at which the absorbent articles and/or packages are diverted to the robotic inspection station 120, etc.).

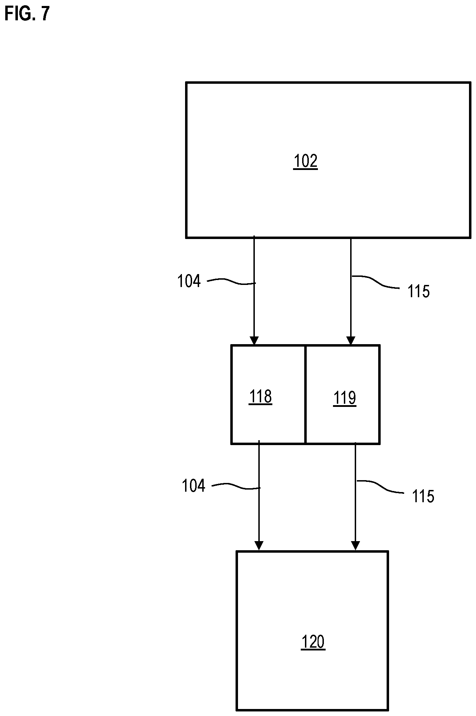

One or more repositories that are used to divert the absorbent articles 104 and/or packages 115 to the robotic inspection station 120 are now discussed. FIG. 7 is an example according to various aspects illustrating a block diagram of repositories 118, 119 for absorbent articles 104 and packages 115 between the absorbent article manufacturing lines 102 and robotic inspection station 120 of FIG. 1. As shown in FIG. 7, in some aspects of the disclosure the diverted absorbent articles 104 and the diverted packages 115 each have their own respective repository 118, 119. However, in other aspects the diverted absorbent articles 104 and packages 115 use the same repository that is upstream of the robotic inspection station 120. In one example aspect, a separate repository 119 is used for the diverted packages 115 in order to move the diverted packages 115 to a different inspection unit 122, 124, 126 that can be used to open the package 115 in order to access the discrete absorbent articles 104 within the package 115 and/or to inspect one or more parameters of the package 115 (e.g., presence of holes, test for strength, etc.). The repositories 118, 119 advantageously provide flexibility in terms of the rate of inspection of the diverted absorbent articles 104 and/or packages 115 so that it need not be at least equal to the rate of manufacturing of the absorbent articles 104 and/or packages 115.

One example aspect of an absorbent article manufacturing line will now be discussed. FIG. 8 is an example according to various aspects illustrating a block diagram of a system 100' for making absorbent articles on an absorbent article manufacturing line 102'. The system 100' is similar to the system 100 previously described, with the exception of the features discussed herein.

As shown in FIG. 8, in one aspect of the disclosure the absorbent article manufacturing line 102' is located on a first or ground floor of a manufacturing facility and the robotic inspection station 120' is located on a second floor 142 of the manufacturing facility. The stream 112' of discrete absorbent articles 104 move (from right to left viewing FIG. 8) to a pad diverter 182 which diverts some of the absorbent articles 104 to the robotic inspection station 120' on the second floor 142 of the manufacturing facility (e.g., using a conveyor 133). For those discrete absorbent articles 104 in the stream 112' which are not diverted, they are packaged using the packaging apparatus 114 after which some of the packages 115 are diverted up to the robotic inspection station 120' with a bag diverter 184 (e.g., using a conveyor 137). In an example aspect, the pad diverter 182 and bag diverter 184 include respective conveyors 133, 137 or other similar mechanism which can be used to divert the absorbent articles and packages to the robotic inspection station 120' on the second floor 142.

In an aspect of the disclosure, a label printer 131 is provided at the robotic inspection station 120' to print one or more labels, such as the label 151 with the product code 152 and/or the label of the characteristic 130 with the marking 132 to indicate the characteristic 130. Additionally, in another aspect of the disclosure, an additional component 135 is provided that performs one or more of pad removal, robotic post-use inspection and retaining of sample storage.

In one aspect of the disclosure, a first inspection unit 122' of the robotic inspection station 120' is an initial inspection unit. In one example aspect of the disclosure, the initial inspection unit can measure one or more of: geometric dimensions that cannot be measured online; a total weight of the discrete absorbent article; an imaging device 136 for inspection of contamination, extraneous material, etc.; and an odor check. In other aspects, the initial inspection units performs a presence of a component test and/or an absence of a component test. In another aspect, components of the absorbent articles may be diverted for testing (e.g., dimensions, weight) prior to being added to the absorbent articles. For example, an absorbent core, ear, or waistband may be diverted for testing.

Specifically, upon arrival at the first robotic inspection station 122', the product may be placed under a camera, and the unique pad code that was printed in the converter would be read by a camera system. This would enable to confirm readability of the pad code and enable to identify the exact product and its relation to certain multi-up processing stations in the line. Consecutive tests performed in the robotic test stations may be referenced to the specific pad code. Furthermore, the product may be opened up using a series of vacuum or mechanical gripping devices and presented to the camera system for an inside and outside inspection of contamination, decolorization or extraneous materials that would be a noticeable defect if shipped in that condition to the user of the product. If any defects are determined, an alert for the machine operator may be provided, and the product may be presented to the machine operator for a more detailed manual inspection and to allow elimination of the source of the defect.

Certain product dimensions may be inspected next. While many product dimensions may be inspected using cameras while the product is made, specific focus of the robotic inspection may be on measurements that can only be performed after the product is cut from the continuous stream of connected products into individual articles. For example, the distance from a side seam to the edge of the product, that is created by the final knife of the machine can only be measured after the stream of products is cut into individual articles, as the final knife creates the edge of the product. If, for example, the distance from side seam to product edge is assessed as out of specification, or trending towards out of specification, the information may be fed back to the machine controller and the machine may self-adjust the phasing of the final knife by accelerating or decelerating the corresponding drive motor.

Following initial dimensional inspections, the product may be placed on a scale to determine total product weight. Considering proper geometric dimensions of nonwoven and film materials that may have been measured while or after the absorbent article was made, and consumption of proper amounts of hot melt adhesives that can be derived off motor pumps the total pad weight may be used as an indicator for the amount of gravimetrically dosed materials such as superabsorber. If the total pad weight is off target, the product may be subjected a secondary series of additional robotic tests. For example, the product may be submerged in a tank of water or synthetic urine to determine total saturated weight as a proxy for absorptivity. Alternatively, an alert may be triggered so the machine operator can initiate a series of manual troubleshooting actions to identify the root cause of the out of specification measurement.

In one aspect of the disclosure, a second inspection unit 124' of the robotic inspection station 120' is a robot product performance test unit. In one example aspect of the disclosure, the robot product performance test unit can measure one or more of: leakage, initial fit/sustained fit, skin marking, fuzz and gel on skin testing.

Following the initial inspection station, the product may be moved to a robotic performance test unit 124', where the diaper or pant may be applied by the robot to an elastic mannequin torso. The torso, with the diaper or pant applied, may be inspected for initial fit, using camera measurement, and consecutively may undergo a series of motion profiles, executed by a robot. Changes in diaper or pant fit may be observed by the camera system, be recorded and provide information about the anticipated performance of the diaper or pant when used. Additionally, the diaper or pant may be loaded with synthetic urine or BM and leakage of the diaper or pant may be observed. If the results of the investigation are not satisfactory, adjustments to the making process may be performed. For example, elastics may be tensioned more tightly if it is observed that the product on the torso slips down more than a predetermined allowable amount. Confirmation of good performance of the product testing can be used as signal to continue operation of the production line without any process adjustments.

Alternatively, following the initial inspection station, the second test unit 124' may be bypassed and the product may be moved to a third test unit 126' where a robotic end-effector equipped with load cells and pressure sensors may be inserted into the pant article. Elastic components of the pant such as the belt, the waist band or the elastic cuff may be tested in various regions of the product. The testing may involve cycling the product through a sequence of opening/closing motions, while the force response of the elastics is being recorded during the loading and unloading cycles. If out of specification measurements are observed, the machine control unit may self-adjust the process by tensioning the elastics in the machine more tightly or more loosely to reestablish good quality. Following a series of non-destructive elastic force measurements other measurements, for example destructive tensile testing of the side seam force may be executed by the same robotic test station. For example, if the side seam tears at a force measurement below spec, the machine may self-adjust the bonding pressure or temperature applied to form the side seam. Upon completion of the destructive test, the test product may be discarded. It is important to notice that certain inspections, such as camera inspections or non-destructive force testing can be performed consecutively using the same article, while other tests including destructive force testing may render the article unusable for further tests. In one aspect of the disclosure, the third inspection unit 126' of the robotic inspection station 120' is a pad force response measurement unit. In one example aspect of the disclosure, the pad force response measurement unit performs destructive testing. In an example aspect, the pad force response measurement unit can measure one or more of: force to failure (e.g., relevant for product integrity and application); interim data points on stress-strain behavior (e.g., before failure); constant displacement and force decay; and product removal force. In still other aspects, the pad force response measurement units performs one or more of: a pull test, a bend test, a sufficiency of force test, an elastic force test, a bond strength test, a tensile test, a weight test, and a dimensional measurement test. In one example aspect, the pad force response unit performs one or more of: American Society for Testing and Materials (ASTM) D 882 "Standard Test Method for Tensile Properties of thin Plastic Sheeting"; 3 point bend ASTM D 790; and ASTM D 76 Standard specification for tensile testing machines for textiles.

As shown in FIG. 8, in some aspects a manual inspection 186 is also performed on the diverted absorbent article 104 and/or package 115 by an operator of the manufacturing line 102'.

In some aspects of the disclosure, the same diverted absorbent article 104 has inspections performed by multiple units including two or more of the initial inspection unit, the robot product performance test and the pad force response measurement unit.

In aspects of the disclosure, the diverted absorbent article 104 is aged (e.g., either in or outside the package 115) for a while prior to undergoing the inspection by the robotic inspection station 120'. In an example aspect, the diverted absorbent article 104 and/or package 115 remain at the respective repository 118, 119 for a predetermined time (e.g., input to the controller 101 using an input device, such as the display 108 or display 507 in the mobile terminal of FIG. 12).

In an aspect of the disclosure, the system 100' includes the controller 101 that receives the data from the inspection units 122', 124', 126'. In some aspects, the controller 101 performs pattern recognition on the data received from the inspection units 122', 124', 126' to identify patterns or trends and provide a diagnosis. In one example aspect, if the controller 101 receives data from the pad force response measurement unit 126' indicating a trend or pattern that a certain layer or component of the discrete absorbent articles 104 fails in destructive testing, the controller 101 can provide a diagnosis such as an identification of a component in the absorbent article manufacturing line 102' that may be not operating properly. In this example aspect, the controller 101 can transmit a signal to the interface (e.g., display) 108 at the absorbent article manufacturing line 102' to communicate to an operator of the manufacturing line that identifies the component that may not be operating correctly. In this example aspect, the operator may then investigate to ensure that the component of the manufacturing line 102' is operating correctly and initiate maintenance or repair operations if required.

In some aspects, the memory 172 of the controller 101 has stored data regarding the values of various parameters (e.g.2, temperature, pressure, etc.) of the components of the absorbent article manufacturing line 102'. In these aspects of the disclosure, upon identifying a trend or pattern in the data from the inspection units 122', 124', 126' as well as data (e.g., product code) that identifies a specific day/time and a specific component of the manufacturing line 102' used to make the absorbent article, the controller 101 assesses the data indicating the parameter values of this specific component at the specific time/day stored in the memory 172. In the event that these parameter values of the specific component are outside a recommended range, the controller 101 transmits a signal to output on the interface (e.g., display) 108 that the specific component should be investigated and/or repaired if necessary.

### Method

A method for making absorbent articles on an absorbent article manufacturing line will now be discussed. FIG. 9 is an example according to various aspects illustrating a flow chart depicting steps of a method 200 for making absorbent articles 104 on an absorbent article manufacturing line 102. Although steps are shown in FIG. 9 as integral blocks in a particular order, in other aspects, one or more steps or portions thereof are performed in a different order or overlapping in time, in series or in parallel, or are omitted or one more additional steps are added.

In step 202, the continuous substrate 110 is advanced. In one aspect, the controller 101 transmits a signal to a transport mechanism (e.g., conveyor) to advance the continuous substrate 110. In an example aspect, in step 202 the continuous substrate 110 is advanced from a first position to a second position along the absorbent article manufacturing line 102 such that additional parts and/or substrates can be added to the continuous substrate 110 at the second position.

In step 204, additional parts and/or substrates are added to the continuous substrate 110 to form a continuous length of absorbent articles. In an example aspect, in step 204 the controller 101 transmits a signal to one or more components on the manufacturing line 102 to add the parts and/or substrates to the continuous substrate 110.

In step 206, the continuous length of absorbent articles from step 204 is cut into separate discrete absorbent articles 104. This results in a stream 112 of discrete absorbent articles. In an example aspect, in step 206 a cutting device is used to cut the continuous length of absorbent articles into the discrete absorbent articles 104. In an example aspect, in step 206 the cutting device is used to cut the discrete absorbent articles 104 based on one or more desired dimensions (e.g., length of the discrete absorbent article 104 between adjacent cuts in the continuous length of absorbent articles).

In step 208, the stream 112 of discrete absorbent articles 104 from step 206 is advanced. In one aspect, in step 208 the controller 101 transmits a signal to transport mechanism (e.g., conveyor) to advance the stream 112. In an example aspect, in step 208 the stream 112 is advanced to a location of a transport mechanism (e.g., secondary conveyor, air tunnel, etc.) used to divert absorbent articles 104 to the robotic inspection station 120.

In step 210, a decision is made whether to divert the discrete absorbent articles 104 in the stream 112. A positive decision in step 210 results in the method moving to step 212. A negative decision in step 210 results in the method moving to step 214. In some aspects, in step 210 the decision is collective for a group of discrete absorbent articles 104. In other aspects, in step 210 the decision is for each discrete absorbent article 104 in the stream 112. In one aspect, in step 210 the controller 101 determines whether to divert the discrete absorbent article 104 based on data stored in the memory 172 indicating a desired rate of diversion (e.g., number of diverted absorbent articles per unit time, ratio of diverted absorbent articles to non-diverted absorbent articles, etc.). In one example aspect, if no discrete absorbent articles 104 have been diverted for the past 5 minutes and the desired rate of diversion is 1 absorbent article 104 per 5 minutes, then the next discrete absorbent article 104 is diverted. In an aspect, in order to divert the discrete absorbent article 104 in step 210, the controller 101 transmits a signal to the transport mechanism for diversion (e.g., secondary conveyor, air tunnel, etc.) to divert the discrete absorbent article 104 to the robotic inspection station 120.

In some aspects, in step 210 the discrete absorbent article 104 is diverted from the stream 112 to the repository 118 prior to being subsequently transported to the robotic inspection station 120. In an example aspect, in step 210 the diverted discrete absorbent article 104 remains at the repository 118 for a predetermined time (e.g., fixed time) or for a sufficient time until the robotic inspection station 120 has available space to inspect the diverted discrete absorbent article 104. In this example aspect, the diverted discrete absorbent article 104 remains at the repository 118 while the robotic inspection station 120 is at full capacity.

In step 212, the diverted discrete absorbent article 104 from step 210 is inspected by the robotic inspection station 120. In one aspect, in step 212 the diverted discrete absorbent article 104 undergoes one or more inspection tests at one or more of the inspection units 122, 124, 126. In step 212, data is generated by the one or more inspection units 122, 124, 126 and transmitted to the controller 101.

In step 214, after deciding in block 212 not to divert the discrete absorbent article 104, the discrete absorbent article 104 is advanced to the packaging apparatus 114. In one aspect of the disclosure, the discrete absorbent article 104 along with other discrete absorbent articles are packaged into a package 115 by the packaging apparatus 114.

In step 216, a decision is made whether to divert the package 115 of the multiple discrete absorbent articles 104 formed in step 214. This decision in step 216 is similar to the decision in step 210 as it is based on data stored in a memory 172 of the controller 101 indicating a desired rate of diversion (e.g., number of diverted packages per unit time, ratio of diverted packages to non-diverted packages, etc.). A positive decision in step 216 moves the method 200 to step 218 whereas a negative decision in step 216 moves the method 200 to step 220.

In step 218, the diverted package 115 from step 216 is inspected by the robotic inspection station 120. In one aspect, in step 218 the diverted package 115 undergoes one or more inspections at one or more of the inspection units 122, 124, 126. In some aspects, in step 218 the package 115 itself is inspected by the one or more of the inspection units 122, 124, 126 using one or more of the tests previously discussed that are performed on the discrete absorbent article 104. In other aspects, in step 218 the package 115 is discarded and the multiple discrete absorbent articles 104 that were within the package 115 are inspected by the one or more inspection units 122, 124, 126. In step 218, data is generated by the one or more inspection units 122, 124, 126 and transmitted to the controller 101.

In step 220, after deciding in block 216 not to divert the package 115, the package 115 is advanced to the end of the manufacturing line 180. In an example aspect, the package 115 is subsequently transported from the end of the manufacturing line 180 to various retail outlets.

In step 222 data is generated based on the inspection steps 212, 218. In one aspect, this data is generated by the one or more inspection units 122, 124, 126 and transmitted to the controller 101. In one example aspect, this data includes identifying data (e.g., product code) of the diverted absorbent article 104 and/or package 115 that communicate one or more identifying indicia of the diverted absorbent article 104 and/or package 115 (e.g., manufacturing line 102 where it was made, time and date it was made, identification of one or more components on the manufacturing line 102 that made it, etc.). In another example aspect, this data includes inspection data such as force measurement data (e.g., based on destructive testing including one or more of the pull test, the bend test, the sufficiency of force test, the elastic force test, the bond strength test and the tensile test); weight measurement data (e.g., weight of the diverted absorbent article and/or package); dimension data (e.g., dimensions of one or more components of the diverted absorbent article and/or package); image data (e.g., captured by the imaging device of the inspection units); positive aspect data (e.g., data indicating the presence of one or more characteristics 130 of the diverted absorbent article or package); and negative aspect data (e.g., data indicating the lack of presence of one or more characteristics 130 of the diverted absorbent article or package).

In an aspect, in step 222 the controller 101 receives the data from the robotic inspection station 120 and compares the data with data stored in the memory 172. In one example aspect, in step 222 the controller 101 compares the force measurement data with data stored in the memory 172 indicating an acceptable range of the force measurement data (e.g., for the discrete absorbent article 104 and/or package 115). In one example aspect, in step 222 the controller 101 compares the dimension data with data stored in the memory 172 indicating an acceptable range of the dimension data (for the discrete absorbent article 104 and/or package 115 including the identified characteristic 130 thereof). In one example aspect, in step 222 the controller 101 compares the positive aspect data with data stored in the memory 172 indicating whether the identified characteristic 130 with the positive aspect data should be present in the diverted absorbent article 104 and/or package 115. In one example aspect, in step 222 the controller 101 compares the negative aspect data with data stored in the memory 172 indicating whether the identified characteristic 130 with the negative aspect data should not be present in the diverted absorbent article 104 and/or package 115.

For purposes of step 222, the controller 101 identifies a positive inspection result for the diverted discrete absorbent article 104 and/or package 115 based on whether the received data from the robotic inspection station 120 conforms with the data stored in the memory 172 (e.g., the force measurement data is within the acceptable range of the force measurement data stored in the memory 172). For purposes of step 222, the controller 101 identifies a negative inspection result for the diverted discrete absorbent article 104 and/or package 115 based on whether the received data from the robotic inspection station 120 does not conform with the data stored in the memory 172 (e.g., positive aspect data indicating holes in a diverted absorbent article 104 should not be present in the diverted absorbent article 104 according to the positive aspect data stored in the memory 172).

In step 224 one or more actions are performed based on the generated data in step 222. In an aspect of the disclosure, in step 224 the controller 101 varies the desired rate of diversion for the discrete absorbent articles 104 and/or packages 115 based on the generated data in step 222. In one example aspect, in step 224 where the controller 101 identifies a trend of an increasing rate of positive inspection results and/or a decreasing rate of negative inspection results for the diverted absorbent articles 104 and/or packages 115 the controller 101 transmits a signal to reduce the desired rate of diversion (e.g., stored in the memory 172). In another example aspect, in step 224 where the controller 101 identifies a trend of a decreasing rate of positive inspection results and/or an increasing rate of negative inspection results for the diverted absorbent articles 104 and/or packages 115 the controller 101 transmits a signal to increase the desired rate of diversion (e.g., stored in the memory 172).

In other aspects, in step 224 the controller 101 transmits a signal to an output device (e.g., interface display 108) at the absorbent article manufacturing line 102 to alert an operator of the manufacturing line with access to the display 108 (e.g., mobile device of the operator). In some aspects, this alert is an electronic notification that can be one or more of visual, audible and/or tactile. In an example aspect, in step 224 the controller transmits a signal to the specific display 108b of the specific absorbent article manufacturing line 102b that was identified in step 222 as the manufacturing line which manufactured the diverted absorbent article 104 and/or package 115 with the negative inspection result (e.g., using the product code 152). This advantageously permits the operator of the specific manufacturing line 102b to take further action (e.g., shut down the specific manufacturing line 102b, investigate whether one or more components of the manufacturing line 102b require repair or maintenance, adjust one or more parameters of the manufacturing line 102, etc.).

In still other aspects, the controller 101 stores parameter values of the components of each absorbent article manufacturing line 102 during the manufacture of each diverted absorbent article 104 and/or package 115 and in step 224 the controller 101 is configured to transmit the signal to the display 108 to output the parameter values of the specific components from the specific manufacturing line 102b responsible for a negative inspection result. This advantageously permits the operator of the specific manufacturing line 102b to observe the parameter values of the specific components of the specific manufacturing line 102b responsible for the negative inspection result and thus take further action (e.g., determine whether the parameter values on the display are outside an acceptable range of the parameter values and perform any further remedial action such as stop the manufacturing line 102b and/or inspect and repair the specific component).

In still other aspects, in step 224 the controller 101 takes additional action such as adding the diverted absorbent article 104 with the positive inspection result back into the absorbent article manufacturing line 102 (e.g., at the packaging apparatus 114 to package the absorbent article into the package 115). This advantageously reduces absorbent article scrap in the absorbent article manufacturing line 102.

In step 226, a determination is made whether additional absorbent articles 104 and/or packages 115 are present which have yet to be considered for diversion to the robotic inspection station 120. If the result of this determination is affirmative, the method proceeds back to step 208. If the result of this determination is negative, the method ends.

### Hardware

FIG. 10 is a block diagram that illustrates a computer system 300 upon which an embodiment of the invention may be implemented. Computer system 300 includes a communication mechanism such as a bus 310 for passing information between other internal and external components of the computer system 300. Information is represented as physical signals of a measurable phenomenon, typically electric voltages, but including, in other embodiments, such phenomena as magnetic, electromagnetic, pressure, chemical, molecular atomic and quantum interactions. For example, north and south magnetic fields, or a zero and non-zero electric voltage, represent two states (0, 1) of a binary digit (bit). Other phenomena can represent digits of a higher base. A superposition of multiple simultaneous quantum states before measurement represents a quantum bit (qubit). A sequence of one or more digits constitutes digital data that is used to represent a number or code for a character. In some embodiments, information called analog data is represented by a near continuum of measurable values within a particular range. Computer system 300, or a portion thereof, constitutes a means for performing one or more steps of one or more methods described herein.

A sequence of binary digits constitutes digital data that is used to represent a number or code for a character. A bus 310 includes many parallel conductors of information so that information is transferred quickly among devices coupled to the bus 310. One or more processors 302 for processing information are coupled with the bus 310. A processor 302 performs a set of operations on information. The set of operations include bringing information in from the bus 310 and placing information on the bus 310. The set of operations also typically include comparing two or more units of information, shifting positions of units of information, and combining two or more units of information, such as by addition or multiplication. A sequence of operations to be executed by the processor 302 constitutes computer instructions.

Computer system 300 also includes a memory 304 coupled to bus 310. The memory 304, such as a random access memory (RAM) or other dynamic storage device, stores information including computer instructions. Dynamic memory allows information stored therein to be changed by the computer system 300. RAM allows a unit of information stored at a location called a memory address to be stored and retrieved independently of information at neighboring addresses. The memory 304 is also used by the processor 302 to store temporary values during execution of computer instructions. The computer system 300 also includes a read only memory (ROM) 306 or other static storage device coupled to the bus 310 for storing static information, including instructions, that is not changed by the computer system 300. Also coupled to bus 310 is a non-volatile (persistent) storage device 308, such as a magnetic disk or optical disk, for storing information, including instructions, that persists even when the computer system 300 is turned off or otherwise loses power.

Information, including instructions, is provided to the bus 310 for use by the processor from an external input device 312, such as a keyboard containing alphanumeric keys operated by a human user, or a sensor. A sensor detects conditions in its vicinity and transforms those detections into signals compatible with the signals used to represent information in computer system 300. Other external devices coupled to bus 310, used primarily for interacting with humans, include a display device 314, such as a cathode ray tube (CRT) or a liquid crystal display (LCD), for presenting images, and a pointing device 316, such as a mouse or a trackball or cursor direction keys, for controlling a position of a small cursor image presented on the display 314 and issuing commands associated with graphical elements presented on the display 314.

In the illustrated embodiment, special purpose hardware, such as an application specific integrated circuit (IC) 320, is coupled to bus 310. The special purpose hardware is configured to perform operations not performed by processor 302 quickly enough for special purposes. Examples of application specific ICs include graphics accelerator cards for generating images for display 314, cryptographic boards for encrypting and decrypting messages sent over a network, speech recognition, and interfaces to special external devices, such as robotic arms and medical scanning equipment that repeatedly perform some complex sequence of operations that are more efficiently implemented in hardware.

Computer system 300 also includes one or more instances of a communications interface 370 coupled to bus 310. Communication interface 370 provides a two-way communication coupling to a variety of external devices that operate with their own processors, such as printers, scanners and external disks. In general the coupling is with a network link 378 that is connected to a local network 380 to which a variety of external devices with their own processors are connected. For example, communication interface 370 may be a parallel port or a serial port or a universal serial bus (USB) port on a personal computer. In some embodiments, communications interface 370 is an integrated services digital network (ISDN) card or a digital subscriber line (DSL) card or a telephone modem that provides an information communication connection to a corresponding type of telephone line. In some embodiments, a communication interface 370 is a cable modem that converts signals on bus 310 into signals for a communication connection over a coaxial cable or into optical signals for a communication connection over a fiber optic cable. As another example, communications interface 370 may be a local area network (LAN) card to provide a data communication connection to a compatible LAN, such as Ethernet. Wireless links may also be implemented. Carrier waves, such as acoustic waves and electromagnetic waves, including radio, optical and infrared waves travel through space without wires or cables. Signals include man-made variations in amplitude, frequency, phase, polarization or other physical properties of carrier waves. For wireless links, the communications interface 370 sends and receives electrical, acoustic or electromagnetic signals, including infrared and optical signals, that carry information streams, such as digital data.

The term computer-readable medium is used herein to refer to any medium that participates in providing information to processor 302, including instructions for execution. Such a medium may take many forms, including, but not limited to, non-volatile media, volatile media and transmission media. Non-volatile media include, for example, optical or magnetic disks, such as storage device 308. Volatile media include, for example, dynamic memory 304. Transmission media include, for example, coaxial cables, copper wire, fiber optic cables, and waves that travel through space without wires or cables, such as acoustic waves and electromagnetic waves, including radio, optical and infrared waves. The term computer-readable storage medium is used herein to refer to any medium that participates in providing information to processor 302, except for transmission media.

Common forms of computer-readable media include, for example, a floppy disk, a flexible disk, a hard disk, a magnetic tape, or any other magnetic medium, a compact disk ROM (CD-ROM), a digital video disk (DVD) or any other optical medium, punch cards, paper tape, or any other physical medium with patterns of holes, a RAM, a programmable ROM (PROM), an erasable PROM (EPROM), a FLASH-EPROM, or any other memory chip or cartridge, a carrier wave, or any other medium from which a computer can read. The term non-transitory computer-readable storage medium is used herein to refer to any medium that participates in providing information to processor 302, except for carrier waves and other signals.

Logic encoded in one or more tangible media includes one or both of processor instructions on a computer-readable storage media and special purpose hardware, such as ASIC *320.

Network link 378 typically provides information communication through one or more networks to other devices that use or process the information. For example, network link 378 may provide a connection through local network 380 to a host computer 382 or to equipment 384 operated by an Internet Service Provider (ISP). ISP equipment 384 in turn provides data communication services through the public, world-wide packet-switching communication network of networks now commonly referred to as the Internet 390. A computer called a server 392 connected to the Internet provides a service in response to information received over the Internet. For example, server 392 provides information representing video data for presentation at display 314.

The invention is related to the use of computer system 300 for implementing the techniques described herein. According to one embodiment of the invention, those techniques are performed by computer system 300 in response to processor 302 executing one or more sequences of one or more instructions contained in memory 304. Such instructions, also called software and program code, may be read into memory 304 from another computer-readable medium such as storage device 308. Execution of the sequences of instructions contained in memory 304 causes processor 302 to perform the method steps described herein. In alternative embodiments, hardware, such as application specific integrated circuit 320, may be used in place of or in combination with software to implement the invention. Thus, embodiments of the invention are not limited to any specific combination of hardware and software.

The signals transmitted over network link 378 and other networks through communications interface 370, carry information to and from computer system 300. Computer system 300 can send and receive information, including program code, through the networks 380, 390 among others, through network link 378 and communications interface 370. In an example using the Internet 390, a server 392 transmits program code for a particular application, requested by a message sent from computer 300, through Internet 390, ISP equipment 384, local network 380 and communications interface 370. The received code may be executed by processor 302 as it is received, or may be stored in storage device 308 or other non-volatile storage for later execution, or both. In this manner, computer system 300 may obtain application program code in the form of a signal on a carrier wave.

Various forms of computer readable media may be involved in carrying one or more sequence of instructions or data or both to processor 302 for execution. For example, instructions and data may initially be carried on a magnetic disk of a remote computer such as host 382. The remote computer loads the instructions and data into its dynamic memory and sends the instructions and data over a telephone line using a modem. A modem local to the computer system 300 receives the instructions and data on a telephone line and uses an infra-red transmitter to convert the instructions and data to a signal on an infra-red a carrier wave serving as the network link 378. An infrared detector serving as communications interface 370 receives the instructions and data carried in the infrared signal and places information representing the instructions and data onto bus 310. Bus 310 carries the information to memory 304 from which processor 302 retrieves and executes the instructions using some of the data sent with the instructions. The instructions and data received in memory 304 may optionally be stored on storage device 308, either before or after execution by the processor 302.

FIG. 11 illustrates a chip set 400 upon which an embodiment of the invention may be implemented. Chip set 400 is programmed to perform one or more steps of a method described herein and includes, for instance, the processor and memory components described with respect to FIG. 5 incorporated in one or more physical packages (e.g., chips). By way of example, a physical package includes an arrangement of one or more materials, components, and/or wires on a structural assembly (e.g., a baseboard) to provide one or more characteristics such as physical strength, conservation of size, and/or limitation of electrical interaction. It is contemplated that in certain embodiments the chip set can be implemented in a single chip. Chip set 400, or a portion thereof, constitutes a means for performing one or more steps of a method described herein.

In one embodiment, the chip set 400 includes a communication mechanism such as a bus 401 for passing information among the components of the chip set 400. A processor 403 has connectivity to the bus 401 to execute instructions and process information stored in, for example, a memory 405. The processor 403 may include one or more processing cores with each core configured to perform independently. A multi-core processor enables multiprocessing within a single physical package. Examples of a multi-core processor include two, four, eight, or greater numbers of processing cores. Alternatively or in addition, the processor 403 may include one or more microprocessors configured in tandem via the bus 401 to enable independent execution of instructions, pipelining, and multithreading. The processor 403 may also be accompanied with one or more specialized components to perform certain processing functions and tasks such as one or more digital signal processors (DSP) 407, or one or more application-specific integrated circuits (ASIC) 409. A DSP 407 typically is configured to process real-world signals (e.g., sound) in real time independently of the processor 403. Similarly, an ASIC 409 can be configured to performed specialized functions not easily performed by a general purposed processor. Other specialized components to aid in performing the inventive functions described herein include one or more field programmable gate arrays (FPGA) (not shown), one or more controllers (not shown), or one or more other special-purpose computer chips.

The processor 403 and accompanying components have connectivity to the memory 405 via the bus 401. The memory 405 includes both dynamic memory (e.g., RAM, magnetic disk, writable optical disk, etc.) and static memory (e.g., ROM, CD-ROM, etc.) for storing executable instructions that when executed perform one or more steps of a method described herein. The memory 405 also stores the data associated with or generated by the execution of one or more steps of the methods described herein.

FIG. 12 is a diagram of exemplary components of a mobile terminal 500 (e.g., cell phone handset) for communications, which is capable of operating in the system of FIG. 2, according to one embodiment. In some embodiments, mobile terminal 501, or a portion thereof, constitutes a means for performing one or more steps described herein. Generally, a radio receiver is often defined in terms of front-end and back-end characteristics. The front-end of the receiver encompasses all of the Radio Frequency (RF) circuitry whereas the back-end encompasses all of the base-band processing circuitry. As used in this application, the term "circuitry" refers to both: (1) hardware-only implementations (such as implementations in only analog and/or digital circuitry), and (2) to combinations of circuitry and software (and/or firmware) (such as, if applicable to the particular context, to a combination of processor(s), including digital signal processor(s), software, and memory(ies) that work together to cause an apparatus, such as a mobile phone or server, to perform various functions). This definition of "circuitry" applies to all uses of this term in this application, including in any claims. As a further example, as used in this application and if applicable to the particular context, the term "circuitry" would also cover an implementation of merely a processor (or multiple processors) and its (or their) accompanying software/or firmware. The term "circuitry" would also cover if applicable to the particular context, for example, a baseband integrated circuit or applications processor integrated circuit in a mobile phone or a similar integrated circuit in a cellular network device or other network devices.

Pertinent internal components of the telephone include a Main Control Unit (MCU) 503, a Digital Signal Processor (DSP) 505, and a receiver/transmitter unit including a microphone gain control unit and a speaker gain control unit. A main display unit 507 provides a display to the user in support of various applications and mobile terminal functions that perform or support the steps as described herein. The display 507 includes display circuitry configured to display at least a portion of a user interface of the mobile terminal (e.g., mobile telephone). Additionally, the display 507 and display circuitry are configured to facilitate user control of at least some functions of the mobile terminal. An audio function circuitry 509 includes a microphone 511 and microphone amplifier that amplifies the speech signal output from the microphone 511. The amplified speech signal output from the microphone 511 is fed to a coder/decoder (CODEC) 513.

A radio section 515 amplifies power and converts frequency in order to communicate with a base station, which is included in a mobile communication system, via antenna 517. The power amplifier (PA) 519 and the transmitter/modulation circuitry are operationally responsive to the MCU 503, with an output from the PA 519 coupled to the duplexer 521 or circulator or antenna switch, as known in the art. The PA 519 also couples to a battery interface and power control unit 520.

In use, a user of mobile terminal 501 speaks into the microphone 511 and his or her voice along with any detected background noise is converted into an analog voltage. The analog voltage is then converted into a digital signal through the Analog to Digital Converter (ADC) 523. The control unit 503 routes the digital signal into the DSP 505 for processing therein, such as speech encoding, channel encoding, encrypting, and interleaving. In one embodiment, the processed voice signals are encoded, by units not separately shown, using a cellular transmission protocol such as enhanced data rates for global evolution (EDGE), general packet radio service (GPRS), global system for mobile communications (GSM), Internet protocol multimedia subsystem (IMS), universal mobile telecommunications system (UMTS), etc., as well as any other suitable wireless medium, e.g., microwave access (WiMAX), Long Term Evolution (LTE) networks, code division multiple access (CDMA), wideband code division multiple access (WCDMA), wireless fidelity (WiFi), satellite, and the like, or any combination thereof.

The encoded signals are then routed to an equalizer 525 for compensation of any frequency-dependent impairments that occur during transmission though the air such as phase and amplitude distortion. After equalizing the bit stream, the modulator 527 combines the signal with a RF signal generated in the RF interface 529. The modulator 527 generates a sine wave by way of frequency or phase modulation. In order to prepare the signal for transmission, an up-converter 531 combines the sine wave output from the modulator 527 with another sine wave generated by a synthesizer 533 to achieve the desired frequency of transmission. The signal is then sent through a PA 519 to increase the signal to an appropriate power level. In practical systems, the PA 519 acts as a variable gain amplifier whose gain is controlled by the DSP 505 from information received from a network base station. The signal is then filtered within the duplexer 521 and optionally sent to an antenna coupler 535 to match impedances to provide maximum power transfer. Finally, the signal is transmitted via antenna 517 to a local base station. An automatic gain control (AGC) can be supplied to control the gain of the final stages of the receiver. The signals may be forwarded from there to a remote telephone which may be another cellular telephone, any other mobile phone or a land-line connected to a Public Switched Telephone Network (PSTN), or other telephony networks.

Voice signals transmitted to the mobile terminal 501 are received via antenna 517 and immediately amplified by a low noise amplifier (LNA) 537. A down-converter 539 lowers the carrier frequency while the demodulator 541 strips away the RF leaving only a digital bit stream. The signal then goes through the equalizer 525 and is processed by the DSP 505. A Digital to Analog Converter (DAC) 543 converts the signal and the resulting output is transmitted to the user through the speaker 545, all under control of a Main Control Unit (MCU) 503 which can be implemented as a Central Processing Unit (CPU) (not shown).

The MCU 503 receives various signals including input signals from the keyboard 547. The keyboard 547 and/or the MCU 503 in combination with other user input components (e.g., the microphone 511) comprise a user interface circuitry for managing user input. The MCU 503 runs a user interface software to facilitate user control of at least some functions of the mobile terminal 501 as described herein. The MCU 503 also delivers a display command and a switch command to the display 507 and to the speech output switching controller, respectively. Further, the MCU 503 exchanges information with the DSP 505 and can access an optionally incorporated SIM card 549 and a memory 551. In addition, the MCU 503 executes various control functions required of the terminal. The DSP 505 may, depending upon the implementation, perform any of a variety of conventional digital processing functions on the voice signals. Additionally, DSP 505 determines the background noise level of the local environment from the signals detected by microphone 511 and sets the gain of microphone 511 to a level selected to compensate for the natural tendency of the user of the mobile terminal 501.

The CODEC 513 includes the ADC 523 and DAC 543. The memory 551 stores various data including call incoming tone data and is capable of storing other data including music data received via, e.g., the global Internet. The software module could reside in RAM memory, flash memory, registers, or any other form of writable storage medium known in the art. The memory device 551 may be, but not limited to, a single memory, CD, DVD, ROM, RAM, EEPROM, optical storage, magnetic disk storage, flash memory storage, or any other non-volatile storage medium capable of storing digital data.

An optionally incorporated SIM card 549 carries, for instance, important information, such as the cellular phone number, the carrier supplying service, subscription details, and security information. The SIM card 549 serves primarily to identify the mobile terminal 501 on a radio network. The card 549 also contains a memory for storing a personal telephone number registry, text messages, and user specific mobile terminal settings.

In some embodiments, the mobile terminal 501 includes a digital camera comprising an array of optical detectors, such as charge coupled device (CCD) array 565. The output of the array is image data that is transferred to the MCU for further processing or storage in the memory 551 or both. In the illustrated embodiment, the light impinges on the optical array through a lens 563, such as a pin-hole lens or a material lens made of an optical grade glass or plastic material. In the illustrated embodiment, the mobile terminal 501 includes a light source 561, such as a LED to illuminate a subject for capture by the optical array, e.g., CCD 565. The light source is powered by the battery interface and power control module 520 and controlled by the MCU 503 based on instructions stored or loaded into the MCU 503.

### Examples/Combinations

1. A method of making absorbent articles on an absorbent article manufacturing line, comprising the steps of:
   advancing a continuous substrate;
   adding parts and other substrates to the continuous substrate to form a continuous length of absorbent articles;
   separating discrete absorbent articles from the continuous length of absorbent articles to form a stream of discrete absorbent articles;
   advancing the stream of discrete absorbent articles to a packaging apparatus;
   automatically diverting some absorbent articles from the stream to a robotic inspection station; and
   automatically inspecting the diverted absorbent articles using the robotic inspection station.
2. The method of Paragraph 1, wherein the automatically diverting step comprises automatically diverting between about 1 absorbent article per five minutes of run time to about 1 absorbent article per fifty hours of run time to the robotic inspection station.
3. The method of Paragraph 1 or 2, comprising performing a first test using the robotic inspection station.
4. The method of Paragraph 3, comprising performing a second test using the robotic inspection station.
5. The method of Paragraph 3, wherein the first test comprises a force measurement test or weight measurement test.
6. The method of Paragraph 4, wherein the second test comprises a softness assessment test or perfume presence test.
7. The method of Paragraph 4, wherein the first test or the second test comprises a pull test, a bend test, a presence of a component test, an absence of a component test, a sufficiency of force test, an elastic force test, a bond strength test, a tensile test, a weight test, and a dimensional measurement test.
8. The method of any one of the preceding paragraphs, comprising automatically inspecting one or more characteristics of the discrete absorbent articles using the robotic inspection system.
9. The method of Paragraph 8, wherein the one or more characteristics comprises a detected defect.
10. The method of Paragraph 9, comprising marking the detected defect on the discrete absorbent article.
11. The method of Paragraph 8, wherein the one or more characteristics comprises a positive aspect or a negative aspect of the discrete absorbent articles.
12. The method of Paragraph 8, wherein the one or more characteristics comprises a negative aspect of the discrete absorbent articles.
13. The method of any one of the preceding paragraphs, comprising:
   generating data from the inspecting step; and
   adjusting the absorbent article manufacturing line based on the generated data.
14. The method of Paragraph 13, comprising tracking the data using a quality control system.
15. The method of any one of the preceding paragraphs, wherein the automatically inspecting the discrete absorbent articles step comprises capturing and storing one or more electronic images.
16. The method of any one of the preceding paragraphs, wherein the discrete absorbent articles are automatically diverted to the robotic inspection station upstream of the packaging apparatus, and wherein the discrete absorbent articles are not positioned in a package.
17. The method of any one of the preceding paragraphs, wherein the absorbent articles are automatically diverted to the robotic inspection station downstream of the packaging apparatus, and wherein the discrete absorbent articles are positioned in a package.
18. The method of any one of the preceding paragraphs, wherein the absorbent article manufacturing line and the robotic inspection station are within the same manufacturing facility.
19. The method of any one of the preceding paragraphs, wherein the absorbent article manufacturing line and the robotic inspection station are on the same manufacturing floor.
20. The method of any one of the preceding paragraphs, comprising reducing absorbent article scrap using the robotic inspection system.
21. The method of any one of the preceding paragraphs, comprising printing or labeling a discrete absorbent article using the robotic inspection system.
22. The method of any one of the preceding paragraphs, wherein the robotic inspection system comprises a first inspection unit, a second inspection unit, and a third inspection unit, comprising:
   performing a first inspection using the first inspection unit;
   performing a second inspection using the second inspection unit; and
   performing a third inspection using the third inspection unit;
   wherein the first, second and third inspections are all different inspections.
23. The method of any one of the preceding paragraphs, wherein the automatically inspecting step comprises reading product codes on the discrete absorbent articles and recording the product codes in sequence.
24. The method of any one of the preceding paragraphs, comprising adjusting the rate of the diverting step based on results of the inspecting step.
25. The method of any one of the preceding paragraphs, comprising providing an electronic notification based on results of the inspective step.
26. The method of Paragraph 17, comprising automatically opening the package in which the discrete absorbent articles are positioned.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A method of making absorbent articles on an absorbent article manufacturing line, comprising the steps of:
advancing a continuous substrate;
adding parts and other substrates to the continuous substrate to form a continuous length of absorbent articles;
separating discrete absorbent articles from the continuous length of absorbent articles to form a stream of discrete absorbent articles;
advancing the stream of discrete absorbent articles to a packaging apparatus;
automatically diverting some absorbent articles from the stream to a robotic inspection station;
automatically inspecting the diverted absorbent articles using the robotic inspection station; and
reducing absorbent article scrap using the robotic inspection system.

2. The method of Claim 1, wherein the automatically diverting step comprises automatically diverting between about 1 absorbent article per five minutes of run time to about 1 absorbent article per fifty hours of run time to the robotic inspection station.

3. The method of Claim 1 or 2, comprising performing a first test using the robotic inspection station.

4. The method of Claim 3, comprising performing a second test using the robotic inspection station.

5. The method of Claim 3, wherein the first test comprises a force measurement test or weight measurement test.

6. The method of Claim 4, wherein the second test comprises a softness assessment test or perfume presence test.

7. The method of Claim 4, wherein the first test or the second test comprises a pull test, a bend test, a presence of a component test, an absence of a component test, a sufficiency of force test, an elastic force test, a bond strength test, a tensile test, a weight test, and a dimensional measurement test.

8. The method of any one of the preceding claims, comprising automatically inspecting one or more characteristics of the discrete absorbent articles using the robotic inspection system.

9. The method of Claim 8, wherein the one or more characteristics comprises a detected defect, comprising marking the detected defect on the discrete absorbent article.

10. The method of Claim 8, wherein the one or more characteristics comprises a positive aspect or a negative aspect of the discrete absorbent articles.

11. The method of any one of the preceding claims, comprising:
generating data from the inspecting step;
adjusting the absorbent article manufacturing line based on the generated data; and
tracking the data using a quality control system.

12. The method of any one of the preceding claims, wherein the discrete absorbent articles are automatically diverted to the robotic inspection station upstream of the packaging apparatus, and wherein the discrete absorbent articles are not positioned in a package.

13. The method of any one of the preceding claims, wherein the absorbent articles are automatically diverted to the robotic inspection station downstream of the packaging apparatus, and wherein the discrete absorbent articles are positioned in a package.

14. The method of any one of the preceding claims, wherein the absorbent article manufacturing line and the robotic inspection station are within the same manufacturing facility.

15. The method of any one of the preceding claims, wherein the absorbent article manufacturing line and the robotic inspection station are on the same manufacturing floor.
